# EUROPEAN PATENT SPECIFICATION

(11) **EP 3 207 852 B1**
(45) Date of publication and mention of the grant of the patent: **05.06.2019**
(21) Application number: 16792449.7
(22) Date of filing: 04.04.2016
(51) Int. Cl.: A61B 1/00, A61B 10/04

(54) **RESIN DISTAL END COMPONENT FOR ENDOSCOPE**
DISTALE ENDOSKOP-ENDKOMPONENTE AUS KUNSTSTOFF
COMPOSANT D'EXTRÉMITÉ DISTALE DE RÉSINE POUR ENDOSCOPE

(30) Priority: 08.05.2015 JP 2015095742
(43) Date of publication of application: 23.08.2017
(73) Proprietor: Olympus Corporation, Hachioji-shi, Tokyo 192-8507 (JP)
(72) Inventor: KANEKO, Hiroyuki, Hachioji-shi, Tokyo 192-8507 (JP)
(74) Representative: Schicker, Silvia
(86) International application number: PCT/JP2016/061029
(87) International publication number: WO 2016/181724

(56) References cited:
- JP-A- 2003 235 787
- JP-A- 2006 255 247
- JP-A- 2009 297 420
- US-A1- 2007 249 907
- US-A1- 2013 274 554

## Description

### Technical Field

The present invention relates to a resin distal end component for an endoscope that is provided at a distal end portion of an endoscope and is formed of two kinds of resin members that are integrated.

### Background Art

An endoscope has been widely used in a medical field and in an industrial field. A diagnosis object of the endoscope or an observation object is an inside of a living body, an inside of a plant, and the like. Therefore, a light source that applies light to the diagnosis object or the observation object is necessary for an endoscope apparatus.

A typical endoscope apparatus includes an endoscope and a light source apparatus as an endoscope external apparatus. The light source apparatus includes a light emitting source that emits illumination light, such as a lamp and a light emitting device.

The illumination light emitted from the light emitting source is transmitted through a light guide fiber provided in the endoscope, and passes through an illumination window provided at a distal end of the insertion section, thereby being applied to the observation object.

The illumination window is typically water-tightly fixed to a through hole that is provided in an insulation distal end cover. In addition, the distal end cover to which the illumination window is fixed is integrally fixed to a distal end rigid portion made of a metal such as a stainless steel. The distal end rigid portion forms the distal end portion of the insertion section.

U.S. Patent Application Publication No. 2007/249907 A1 discloses an imaging assembly for an endoscope including a transparent distal cap (e.g., made of a plastic material such as clear polycarbonate) positioned at the distal end of the endoscope. A distal face of the transparent cap includes a pair of windows, molded into the distal cap and allowing illumination from an illumination source to reach a tissue to be imaged by the endoscope. An opening is provided between the windows to receive an objective lens assembly for an image sensor. A further opening, serving as an entrance to a working channel of the endoscope, is provided below the opening for the lens assembly. U.S. Patent Application Publication No. 2013/274554 A1 discloses an endoscope having, at its distal end portion, a distal end cover integrally including a first molded portion and a second molded portion. The first molded portion is molded from a transparent first resin member having insulating properties, and is configured to act as an optical portion. On the other hand, the second molded portion is molded from a colored second resin member having insulating properties, and is configured to act as a light blocking portion. Specifically, the first molded portion is exposed and includes at least one illumination window portion provided at its distal end face.

International Publication No. 2011-111242 discloses a method of manufacturing a resin molded product and a resin molded product. The resin molded product is manufactured by integrally molding an optical device and a supporting member supporting the optical device. The optical device is used for an observation optical system of an endoscope and/or an image pickup optical system of a camera.

In Fig. 27, Fig. 35, and other drawings of International Publication No. 2011-111242, a two-color molded product in which an illumination lens is made of a resin for an optical component, and a distal end portion body serving as the supporting member is made of an optically opaque resin such as a black resin, is illustrated. The two-color molded product makes it possible to form the distal end portion of the insertion section without increasing a diameter of the insertion section of the endoscope.

In the above-described two-color molded product that forms the distal end portion of the insertion section of the endoscope, however, a boundary between the illumination lens and the distal end portion body, in other words, a boundary between the transparent resin for the optical component and the optically opaque resin is exposed to the outside. Although the boundary is a fine groove (also referred to as a notch), the notch may become a starting point of crack when the endoscope is repeatedly subjected to high-pressure high-temperature steam sterilization (also referred to as autoclave sterilization).

The present invention is made in consideration of the above-described circumstances, and an object of the present invention is to provide a resin distal end component for an endoscope that is formed through integral molding of a transparent resin for an optical component and a resin different from the transparent resin, and is suitable to be disposed at the distal end portion of the insertion section.

### Disclosure of Invention

### Means for Solving the Problem

A resin distal end component for an endoscope according to an aspect of the present invention includes the features of claim 1. A resin distal end component for an endoscope may include: a transparent resin outer layer portion that is made of a transparent first resin member and forms a surface of a distal end portion of an insertion section; an internal structure portion that is made of a second resin member different from the first resin member, and is provided in tight contact with an inner surface of the transparent resin outer layer portion; a plurality of component fixing holes in which distal-end-side end portions of components provided in the insertion section are respectively disposed, the component fixing holes being provided in the internal structure portion; and lens sections provided in the transparent resin outer layer portion, the lens sections respectively facing distal end surfaces of optical components disposed in the insertion section, the optical components each having a distal-end-side end portion disposed in a corresponding component fixing hole of the component fixing holes.

The present invention makes it possible to realize the resin distal end component for the endoscope that is formed by integral molding of the transparent resin for the optical component and the resin different from the transparent resin and is suitable to be disposed at the distal end portion of the insertion section.

### Brief Description of the Drawings

Fig. 1 is a diagram to explain an endoscope;
Fig. 2A is a front view of a distal end portion of an insertion section;
Fig. 2B is a diagram of the distal end portion of the insertion section including an internal structure portion, as viewed from a front side;
Fig. 3 is a cross-sectional diagram taken along arrow line Y3-Y3 in Fig. 2A;
Fig. 4 is a cross-sectional diagram taken along arrow line Y4-Y4 in Fig. 2A;
Fig. 5A is a diagram illustrating a cross-section taken along arrow line Y5-Y5 similar to the arrow line Y3-Y3 in Fig. 2A, and is a diagram to explain a distal end component that is formed of a transparent resin member and an opaque resin member;
Fig. 5B is a cross-sectional diagram taken along arrow line Y5B-Y5B in Fig. 2A, and is a diagram to explain the distal end component that is formed of the transparent resin member and the opaque resin member;
Fig. 6 is a diagram of the distal end component as viewed from a front side to explain other configuration example of the distal end component; and
Fig. 7 is a cross-sectional diagram taken along arrow line Y7-Y7 in Fig. 6.

### Best Mode for Carrying Out the Invention

Hereinafter, an embodiment of the present invention is described with reference to drawings.

Note that scale sizes of some components are varied in illustration for each component in order to illustrate the components with respective recognizable sizes in the drawings used in the following description. Moreover, the present invention is not limited to the number of components, shapes of the components, a size ratio of the components, and relative positional relationship between the respective components that are illustrated in the respective drawings.

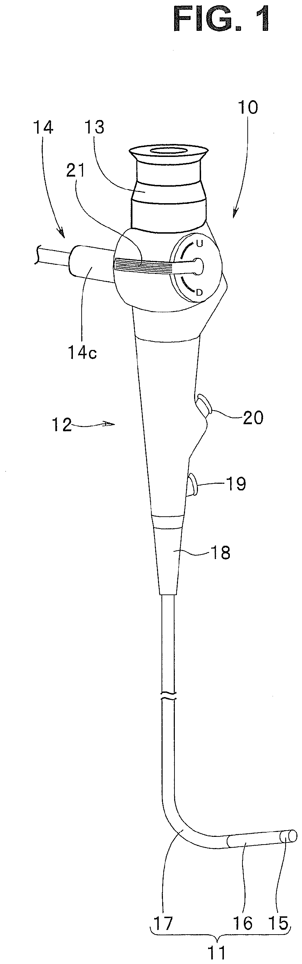

An endoscope 10 illustrated in Fig. 1 is, for example, a nephro-ureteroscope, and is mainly configured by providing an insertion section 11, an operation section 12, and an eyepiece section 13. The eyepiece section 13 is provided on proximal end side of the operation section 12.

A reference numeral 14 denotes a universal cord, and a connector 14c of the universal cord 14 is detachable to a light guide connection opening (not illustrated) that is provided on a side part of the operation section 12.

The insertion section 11 is configured by providing a rigid distal end portion 15, a bending portion 16, and a flexible tube portion 17 that are connected with one another in order from the distal end side of the insertion section 11. The bending portion 16 is so configured as to be bent in a vertical direction. The flexible tube portion 17 is a flexible tube body.

A bend preventing portion 18 having predetermined elastic force is provided on proximal end side of the flexible tube portion 17. The bend preventing portion 18 is so provided as to cover a proximal end portion of the flexible tube portion 17 to prevent buckling of the flexible tube portion 17 and to retain water-tightness between the flexible tube portion 17 and distal end side of the operation section 12.

A water leakage detection pipe sleeve 19, a treatment instrument insertion opening 20, a bending operation lever 21, and the like are provided on the operation section 12. The bending operation lever 21 is rotatable, and the bending portion 16 is bent in the vertical direction through pulling and relaxing of a bending wire (not illustrated) along with rotation operation of the lever 21.

Note that the bending portion 16 may be configured by providing an active bending portion and a passive bending portion. The active bending portion is bent through pulling and relaxing of the bending wire, and the passive bending portion is bent in response to external force. In addition, the endoscope 10 is not limited to a nephro-ureteroscope.

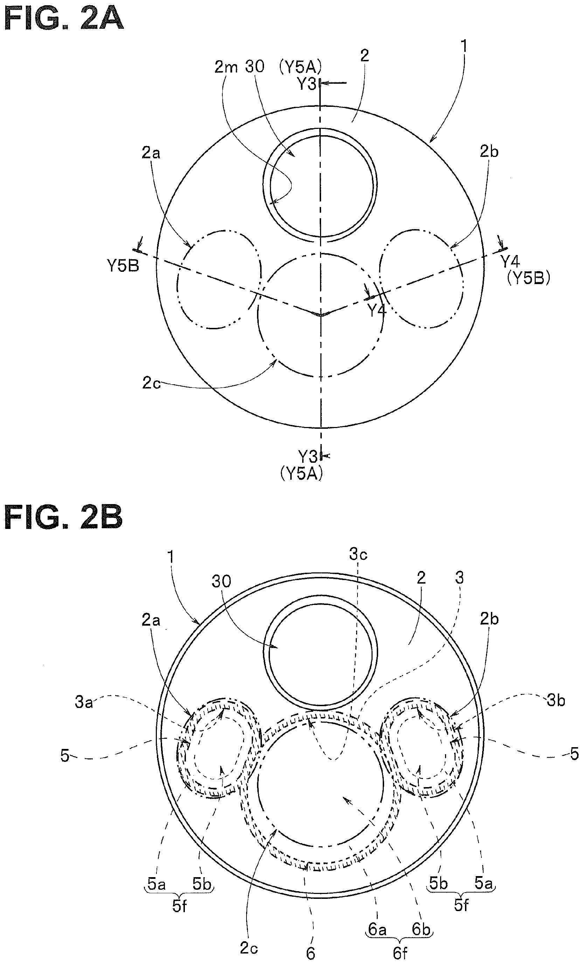

A reference numeral 2 illustrated in Fig. 2A denotes a transparent resin outer layer portion that configures a distalmost end portion of a resin distal end component for the endoscope (hereinafter, abbreviated as the distal end component). The transparent resin outer layer portion 2 is made of a first resin member, and forms a distal end surface of the distal end portion 15. The first resin member is an insulation transparent resin for an optical component.

An opening 2m of a treatment instrument channel hole 30 is provided at a predetermined position of the transparent resin outer layer portion 2 that is a front surface of the distal end component 1. In addition, illumination lens sections 2a and 2b and an observation lens section 2c that are illustrated by alternate long and two short dash lines, are provided at respective predetermined positions of the transparent resin outer layer portion 2.

In the present drawing, the two illumination lens sections 2a and 2b are so provided as to sandwich the observation lens section 2c. The number of the illumination lens sections, however, is not limited to two, and two or more or two or less illumination lens sections may be provided.

As illustrated in Fig. 2B, an internal structure portion 3 is provided on inner side of the transparent resin outer layer portion 2 in the distal end portion 15. The internal structure portion 3 includes through holes 3a, 3b, and 3c in which two illumination optical sections 5 and an observation optical section 6 are respectively disposed.

A reference numeral 5f denotes a distal end surface of each of the illumination optical sections 5, and the distal end surfaces of the illumination optical sections 5 respectively face the illumination lens sections 2a and 2b. A reference numeral 5a denotes a pipe, and a reference numeral 5b denotes a light guide fiber.

In contrast, a reference numeral 6f denotes a distal end surface of the observation optical section 6, and the distal end surface of the observation optical section 6 faces the observation lens section 2c. A reference numeral 6a denotes a first lens frame, and a reference numeral 6b denotes an observation lens.

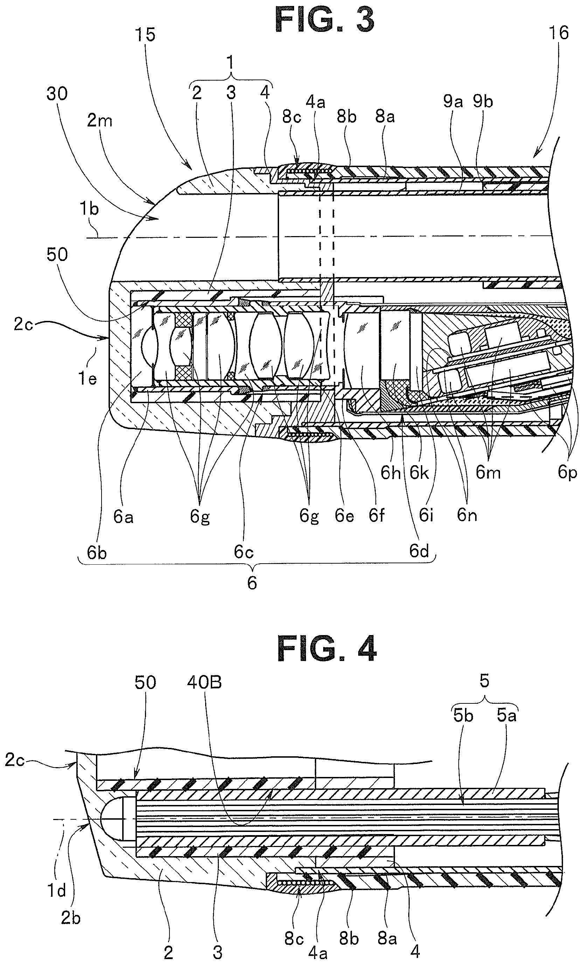

As illustrated in Fig. 3 and Fig. 4, the distal end component 1 is provided at the distal end portion 15.

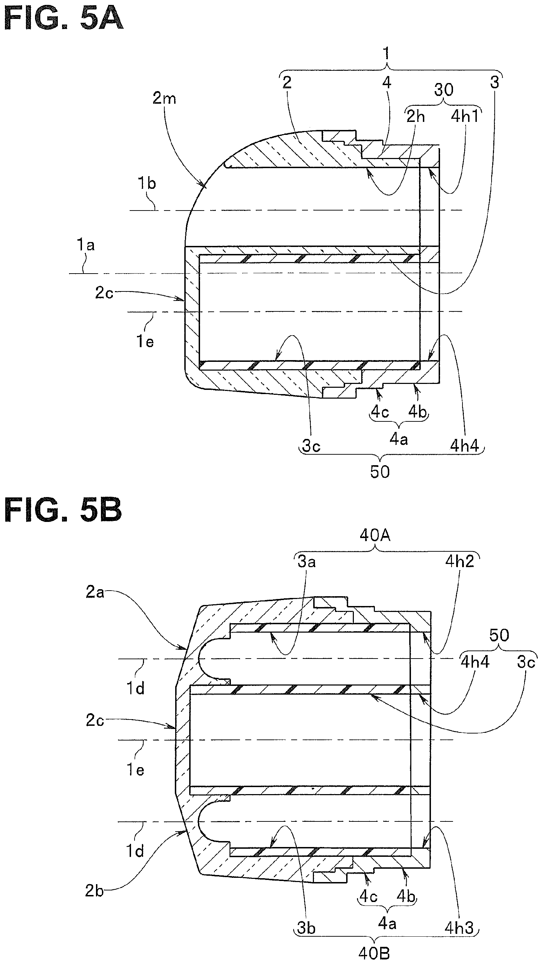

As illustrated in Fig. 5A and Fig. 5B, the distal end component 1 is a substantially cylindrical distal end constituent member that includes the transparent resin outer layer portion 2, the internal structure portion 3, and a coupling portion 4.

The internal structure portion 3 is made of a second resin member that is different from the first resin member. The internal structure portion 3 is provided in tight contact with an inner surface 2i of the transparent resin outer layer portion 2. Further, the internal structure portion 3 is so provided as not to be exposed on the distal end surface of the distal end portion 15.

The second resin member is an optically opaque insulation resin, for example, a black resin. In other words, in the present embodiment, the internal structure portion 3 functions as a light shielding section.

The transparent resin outer layer portion 2 and the internal structure portion 3 are formed as a two-color molded product 7 through integral molding.

The coupling portion 4 is a light shielding member, for example, an annular member made of a stainless steel pipe.

A peripheral step part 4a is provided on an outer peripheral surface on the proximal end side of the coupling portion 4. The peripheral step part 4a includes a first step part 4b and a second step part 4c. For example, the first step part 4b is a step part for a piece, and the second step part 4c is a step part for a rubber.

The distal end of the coupling portion 4 is disposed on an outer peripheral surface on the proximal end side of the transparent resin outer layer portion 2 that is configured as the two-color molded product 7. In the arrangement state, an inner surface of the proximal end part of the coupling portion 4 is disposed to tightly seal a boundary 7a between the internal structure portion 3 and the transparent resin outer layer portion 2 that is exposed on a proximal end surface of the two-color molded product 7.

Further, the coupling portion 4 is integrally fixed to the two-color molded product 7 by, for example, an adhesive, thereby configuring the distal end component 1. Therefore, an outer surface of the distal end component 1 is formed of the transparent resin outer layer portion 2 and the coupling portion 4.

In the distal end component 1, the treatment instrument channel hole 30 that is a through hole, and illumination optics holes 40A and 40B and an observation optics hole 50 that are blind holes are provided.

Each of the treatment instrument channel hole 30, the illumination optics holes 40A and 40B, and the observation optics hole 50 also serves as a component fixing hole.

The treatment instrument channel hole 30 has a first axis 1b that is parallel to a longitudinal axis 1a of the distal end component 1. The treatment instrument channel hole 30 is formed of a through hole 2h and a treatment through hole 4h1. The through hole 2h has the distal end opening 2m provided in the transparent resin outer layer portion 2. The treatment through hole 4h1 is provided in the coupling portion 4.

The first illumination optics hole 40A is provided corresponding to the first illumination lens section 2a, and has a second axis 1c that is parallel to the longitudinal axis la of the distal end component 1. The first illumination optics hole 40A is formed of a first through hole 3a of the internal structure portion 3 and a first illumination optics through hole 4h2. The first illumination optics through hole 4h2 is provided in the coupling portion 4.

The second illumination optics hole 40B is provided corresponding to the second illumination lens section 2b, and has a third axis 1d that is parallel to the longitudinal axis la of the distal end component 1. The second illumination optics hole 40B is formed of a second through hole 3b of the internal structure portion 3 and a second illumination optics through hole 4h3. The second illumination optics through hole 4h3 is provided in the coupling portion 4.

The observation optics hole 50 is provided corresponding to the observation lens section 2c, and has a fourth axis 1e that is parallel to the longitudinal axis 1a of the distal end component 1. The observation optics hole 50 is formed of a third through hole 3c of the internal structure portion 3 and an observation optics through hole 4h3. The observation optics through hole 4h3 is provided in the coupling portion 4.

As illustrated in Fig. 3 and Fig. 4, a distal end bending piece 8a of an unillustrated bending portion set forming the bending portion 6 is disposed at the step part for the piece of the peripheral step part 4a, and a bending rubber 8b is disposed at the step part for the rubber. The bending rubber 8b covers the bending portion set to form the bending portion 16.

A reference numeral 8c denotes a bobbin adhesion fixing portion.

As illustrated in Fig. 4, the distal end portions of the illumination optical sections 5 that are optical components are respectively fixed to the illumination optics holes 40A and 40B.

Each of the illumination optical sections 5 includes a pipe 5a and a light guide fiber 5b. A distal end portion of the light guide fiber 5b is protected by the pipe 5a.

In contrast, as illustrated in Fig. 3, a distal end portion of a tube pipe sleeve 9a that is one of components is fixed to the proximal end side in the treatment instrument channel hole 30. A distal end portion of a channel tube 9b is externally fitted to a proximal end portion of the tube pipe sleeve 9a.

A treatment instrument such as a biopsy needle is guided into the channel tube 9b from the treatment instrument insertion opening 20 illustrated in Fig. 1, is guided into the treatment instrument channel hole 30 through the tube pipe sleeve 9a, and is then guided out from the distal end opening 2m.

A distal end portion of the observation optical section 6 that is one optical component is fixed to the observation optics hole 50.

The observation optical section 6 is mainly configured by having an observation lens 6b, an objective optical system 6c, an image pickup unit 6d, and the like. A reference numeral 6a denotes a first lens frame, a reference numeral 6e denotes a second lens frame, and a reference numeral 6f denotes an image pickup frame.

The observation lens 6b is disposed inside the first lens frame 6a. A plurality of kinds of optical lenses 6g, a spacer ring (not illustrated), a diaphragm (not illustrated), and the like are disposed as optical members inside the second lens frame 6e. A distal end portion of the second lens frame 6e is fixed to the first lens frame 6a. An optical lens 6h that is an optical member, and the like are disposed inside the image pickup frame 6f. A proximal end portion of the second lens frame 6e is fixed to the image pickup frame 6f.

The image pickup unit 6d includes: a solid-state image pickup device 6i such as a CCD and a CMOS; a cover glass 6k that is disposed in front of the image pickup surface; a circuit substrate 6n mounted with a plurality of electronic parts 6m; a signal cable (not illustrated) that is formed of a bundle of a plurality of signal lines 6p each connected to the circuit substrate 6n; and other components.

According to the endoscope 10 having the above-described configuration, when illumination light is emitted from an unillustrated light source apparatus, the illumination light is transmitted by the light guide fiber 5b that is provided in the endoscope 10. The illumination light is then outputted from the illumination optical sections 5 that are respectively disposed in the illumination optics holes 40A and 40B of the distal end component 1 configuring the distal end portion 15 of the insertion section 11.

The illumination light outputted from the distal ends of the respective illumination optical sections 5 is diffused by concave parts provided on lens rear surfaces of the respective illumination lens sections 2a and 2b. The diffused light is applied from lens front surfaces of the respective illumination lens sections 2a and 2b to the inside of a body cavity after passing through the transparent resin outer layer portion 2, thereby illuminating an observation site with appropriate light distribution.

As a result, an optical image of the observation site illuminated with the illumination light passes from the lens front surface of the observation lens section 2c through the transparent resin outer layer portion 2, then passes through the lens rear surface to enter the observation optical section 6, thereby being picked up by the image pickup unit 6d.

As a result, intended optical performance is realized, and an excellent endoscope image is displayed on a screen of an unillustrated display apparatus.

The endoscope 10 after use is sterilized in an autoclave.

In the present invention, the distal end component 1 in which the coupling portion 4 is integrally fixed to the two-color molded product 7 is provided at the distal end portion 15 of the insertion section 11. The two-color molded product 7 is formed of the transparent resin outer layer portion 2 and the internal structure portion 3 through two-color molding. In addition, the outer surface of the distal end component 1 is formed of the coupling portion 4 and the transparent resin outer layer portion 2 that has no boundary between the first resin member and the second resin member because of the two-color molding.

Such a configuration makes it possible to dispose only the transparent resin outer layer portion 2 of the two-color molded product 7 on the outer surface of the distal end component 1. This eliminates crack that occurs from a notch as a starting point when the autoclave sterilization is repeatedly performed. The notch is a fine groove.

In addition, configuring the internal structure portion 3 as the light shielding section makes it possible to surely prevent the illumination light of the illumination optical sections 5 provided in the respective through holes 3a and 3b of the internal structure portion 3, from being outputted to the outside through the internal structure portion 3.

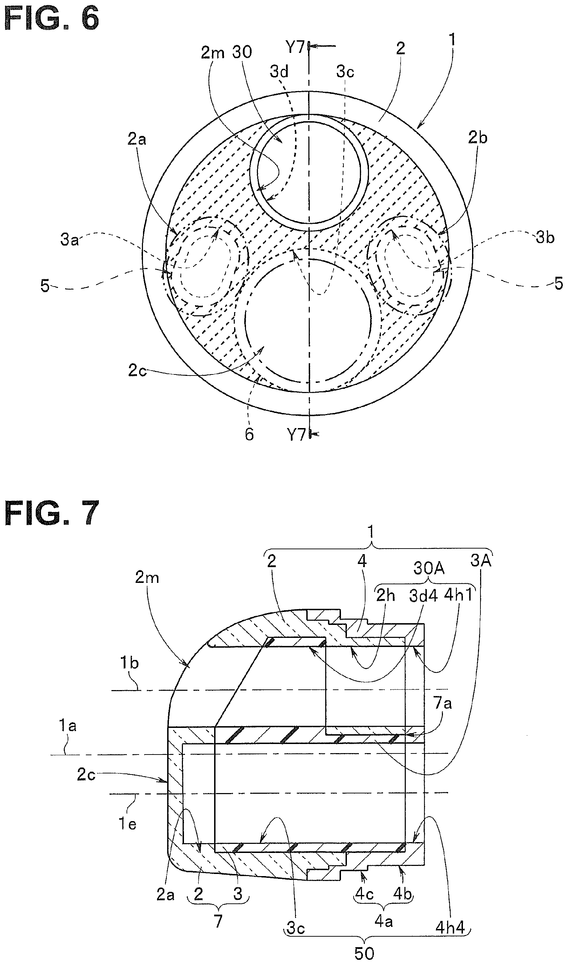

Note that, in the above-described embodiment, the internal structure portion 3 includes the through holes 3a, 3b, and 3c as illustrated in Fig. 2B. As illustrated in Fig. 6 and Fig. 7, however, an internal structure portion 3A that includes a fourth through hole 3d in addition to the through holes 3a, 3b, and 3c may be adopted.

The fourth through hole 3d is a treatment instrument insertion through hole.

An outer shape of the internal structure portion 3 is not limited to the shape in the above-described embodiment. Other configurations of the internal structure portion 3 are similar to the configurations in the above-described embodiment, and the same components are denoted by the same reference numerals as the above-described embodiment and description of such components is omitted.

In the present embodiment, a treatment instrument channel hole 30A is composed of: the through hole 2h that has the distal end opening 2m provided in the transparent resin outer layer portion 2; the fourth through hole 3d of the internal structure portion 3A; and the treatment through hole 4h1 provided in the coupling portion 4.

The configuration makes it possible to provide action and effects similar to the action and effects of the above-described embodiment.

Note that the present invention is not limited only to the above-described embodiments, and may be variously modified without departing from the scope of the claims.

## Claims

1. A resin distal end component (1) for an endoscope (10), the distal end component (1) configuring a distal end portion (15) of an insertion section (11) of the endoscope (10), the distal end component (1) comprising:
a transparent resin outer layer portion (2) being made of a transparent first resin member and forming a distal end surface of the distal end portion (15) of the insertion section (11);
an internal structure portion (3) that is made of a second resin member different from the first resin member, and is provided in tight contact with an inner surface (2i) of the transparent resin outer layer portion (2) without being exposed on the distal end surface,
wherein the transparent resin outer layer portion (2) and the internal structure portion (3) are formed as a two-color molded product (7) through integral molding;
a coupling member (4) integrally fixed to the two-color molded product (7), wherein the distal end of the coupling member (4) is disposed on an outer peripheral surface on the proximal end side of the transparent resin outer layer portion (2), and wherein the entire outer surface of the distal end component (1) is formed of the coupling portion (4) and the transparent resin outer layer portion (2) and has no boundary between the first resin member and the second resin member; a plurality of component fixing holes (40A, 40B, 50) in which distal-end-side end portions of optical components (5, 6) provided in the insertion section (11) are respectively disposed, the component fixing holes (40A, 40B, 50) being provided in the internal structure portion (3); and
lens sections (2a, 2b, 2c) integrally formed in the transparent resin outer layer portion (2) by said integral molding, the lens sections (2a, 2b, 2c) respectively facing distal end surfaces of the optical components (5, 6) disposed in the insertion section (11), the optical components (5, 6) each having a distal-end-side end portion disposed in a corresponding component fixing hole of the component fixing holes (40A, 40B, 50).

2. The resin distal end component (1) for the endoscope (10) according to claim 1, wherein one or more of the plurality of component fixing holes (40A, 40B, 50) are optical component fixing holes (40A, 40B, 50) in which the optical components (5, 6) are respectively disposed, each of the optical components (5, 6) including an optical section that faces a corresponding lens section (2a, 2b, 2c) of the lens sections (2a, 2b, 2c) provided in the transparent resin outer layer portion (2).

3. The resin distal end component (1) for the endoscope (10) according to claim 1, wherein the internal structure portion (3) serves as a light shielding section.

4. The resin distal end component (1) for the endoscope (10) according to claim 3, wherein the coupling portion (4) serves as a light shielding member.

## Patentansprüche

1. Distale Endkomponente aus einem Harz (1) für ein Endoskop (10), wobei die distale Endkomponente (1) einen distalen Endabschnitt (15) eines Einführabschnitts (11) des Endoskops (10) ausbildet, wobei die distale Endkomponente (1) umfasst:
einen Außenschichtabschnitt (2) aus einem transparenten Harz, der aus einem transparenten ersten Harzelement hergestellt ist und eine Distalendfläche des Distalendabschnitts (15) des Einführabschnitts (11) bildet;
einen Innenstrukturabschnitt (3), der aus einem zweiten Harzelement hergestellt ist, das sich von dem ersten Harzelement unterscheidet, und in engem Kontakt mit einer Innenfläche (2i) des Außenschichtabschnitts (2) aus einem transparenten Harz ist, ohne an der Distalendfläche freizuliegen,
wobei der Außenschichtabschnitt (2) aus einem transparenten Harz und der Innenstrukturabschnitt (3) durch integrales Formen als ein zweifarbiges Formteil (7) gebildet sind;
ein Kopplungselement (4), das einstückig an dem zweifarbigen Formteil (7) befestigt ist, wobei das distale Ende des Kopplungselements (4) an einer peripheren Außenfläche auf der Proximalendseite des Außenschichtabschnitts (2) aus einem transparenten Harz angeordnet ist, und wobei die gesamte Außenfläche der Distalendkomponente (1) aus dem Kopplungsabschnitt (4) und dem Außenschichtabschnitt (2) aus einem transparenten Harz gebildet ist und keine Grenze zwischen dem ersten Harzelement und dem zweiten Harzelement aufweist;
eine Mehrzahl von Komponentenbefestigungslöchern (40A, 40B, 50), in denen Distalendseitenendabschnitte von optischen Komponenten (5, 6), die in dem Einführabschnitt (11) vorgesehen sind, jeweils angeordnet sind, wobei die Komponentenbefestigungslöcher (40A, 40B, 50) in dem Innenstrukturabschnitt (3) vorgesehen sind; und
Linsenabschnitte (2a, 2b, 2c), die einstückig in dem Außenschichtabschnitt (2) aus einem transparenten Harz durch das integrale Formen gebildet sind, wobei die Linsenabschnitte (2a, 2b, 2c) jeweils Distalendflächen der optischen Komponenten (5, 6) zugewandt sind, die in dem Einführabschnitt (11) angeordnet sind, wobei die optischen Komponenten (5, 6) jeweils einen Distalendseitenendabschnitt aufweisen, der in einem entsprechenden Komponentenbefestigungsloch der Komponentenbefestigungslöcher (40A, 40B, 50) angeordnet ist.

2. Distale Endkomponente aus einem Harz (1) für ein Endoskop (10) nach Anspruch 1, wobei eines oder mehrere der Mehrzahl von Komponentenbefestigungslöchern (40A, 40B, 50) Befestigungslöcher (40A, 40B, 50) für optische Komponenten sind, in denen die optischen Komponenten (5, 6) jeweils angeordnet sind, wobei jede der optischen Komponenten (5, 6) einen optischen Abschnitt umfasst, der einem entsprechenden Linsenabschnitt (2a, 2b, 2c) der Linsenabschnitte (2a, 2b, 2c) zugewandt ist, die in dem Außenschichtabschnitt (2) aus einem transparenten Harz vorgesehen sind.

3. Distale Endkomponente aus einem Harz (1) für ein Endoskop (10) nach Anspruch 1, wobei der Innenstrukturabschnitt (3) als ein lichtabschirmender Abschnitt dient.

4. Distale Endkomponente aus einem Harz (1) für ein Endoskop (10) nach Anspruch 3, wobei der Kopplungsabschnitt (4) als ein lichtabschirmendes Element dient.

## Revendications

1. Composant d'extrémité distale en résine (1) pour un endoscope (10), le composant d'extrémité distale (1) configurant une partie d'extrémité distale (15) d'une section d'insertion (11) de l'endoscope (10), le composant d'extrémité distale (1) comprenant :
une partie couche externe de résine transparente (2) faite d'un premier élément de résine transparent et formant une surface d'extrémité distale de la partie d'extrémité distale (15) de la section d'insertion (11) ;
une partie structure interne (3) qui est faite d'un second élément de résine différent du premier élément de résine, et est disposée en contact étroit avec une surface interne (2i) de la partie couche externe de résine transparente (2) sans être exposée sur la surface d'extrémité distale,
la partie couche externe de résine transparente (2) et la partie structure interne (3) étant formées en tant que produit moulé à deux couleurs (7) par moulage d'un seul tenant ;
un élément de couplage (4) fixé d'un seul tenant au produit moulé à deux couleurs (7), l'extrémité distale de l'élément de couplage (4) étant disposée sur une surface périphérique externe sur le côté extrémité proximale de la partie couche externe de résine transparente (2), et la surface externe entière du composant d'extrémité distale (1) étant formée de la partie de couplage (4) et de la partie couche externe de résine transparente (2) et n'ayant pas de frontière entre le premier élément de résine et le second élément de résine ;
une pluralité de trous de fixation de composant (40A, 40B, 50) dans lesquels des parties d'extrémité côté extrémité distale de composants optiques (5, 6) prévus dans la partie d'insertion (11) sont respectivement disposées, les trous de fixation de composant (40A, 40B, 50) étant prévus dans la partie structure interne (3) ; et
des sections de lentille (2a, 2b, 2c) formées d'un seul tenant dans la partie couche externe de résine transparente (2) par ledit moulage d'un seul tenant, les sections de lentille (2a, 2b, 2c) faisant respectivement face à des surfaces d'extrémité distale des composants optiques (5, 6) disposés dans la section d'insertion (11), les composants optiques (5, 6) ayant chacun une partie d'extrémité côté extrémité distale disposée dans un trou de fixation de composant correspondant parmi les trous de fixation de composant (40A, 40B, 50).

2. Composant d'extrémité distale en résine (1) pour l'endoscope (10) selon la revendication 1, dans lequel un ou plusieurs parmi la pluralité de trous de fixation de composant (40A, 40B, 50) sont des trous de fixation de composant optique (40A, 40B, 50) dans lesquels les composants optiques (5, 6) sont respectivement disposés, chacun des composants optiques (5, 6) comprenant une section optique qui fait face à une section de lentille correspondante (2a, 2b, 2c) parmi les sections de lentille (2a, 2b, 2c) prévues dans la partie couche externe de résine transparente (2).

3. Composant d'extrémité distale en résine (1) pour l'endoscope (10) selon la revendication 1, dans lequel la partie structure interne (3) sert de section de protection contre la lumière.

4. Composant d'extrémité distale en résine (1) pour l'endoscope (10) selon la revendication 3, dans lequel la partie de couplage (4) sert d'élément de protection contre la lumière.
